(19) **Europäisches Patentamt**
**European Patent Office**
**Office européen des brevets**

(11) **EP 3 575 797 A1**

(12) **EUROPEAN PATENT APPLICATION**

(43) Date of publication:
04.12.2019 Bulletin 2019/49

(51) Int Cl.:
*G01N 33/68* (2006.01)

(21) Application number: 18382384.8

(22) Date of filing: 01.06.2018

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Fundació Centre de Regulació Genòmica**
08003 Barcelona (ES)
• **Universitat Pompeu Fabra**
08002 Barcelona (ES)

(72) Inventors:
• **SABIDO AGUADE, EDUARD**
E-08003 BARCELONA (ES)
• **BORRAS RAMIREZ, EVA**
E-08002 BARCELONA (ES)

(74) Representative: **Moore, Michael Richard et al Keltie LLP**
**No.1 London Bridge**
**London SE1 9BA (GB)**

(54) **IMPROVEMENTS IN MASS SPECTROMETRY**

(57) Disclosed herein are novel methods for analysing a sample using a tandem mass spectrometer. The method comprises executing a data acquisition cycle comprising: isolating precursor ions in a mass-to-charge (m/z) isolation window, fragmenting the precursor ions to generate fragment ions, and mass analysing the fragment ions. Isolating precursor ions in an isolation window comprises pooling precursor ions from N>=2 mass-to-charge isolation sub-windows, wherein the boundaries of a first isolation sub-window are defined as $m/z=[x^1{}_A, x^1{}_B]$ and the boundaries of the second and any subsequent isolation sub-window are defined relative to the boundaries of the first isolation sub-window to capture ions derived from the same molecule but with different charge states. A mass spectrometer configured to implement the methods of the invention is also provided.

Figure 2

EP 3 575 797 A1

**Description**

**Field of the Invention**

This invention relates to methods and apparatus for analysing samples by mass spectrometry. In particular, the invention relates to methods and apparatus for analysing peptides or proteins by tandem mass spectrometry wherein fragment ion signals from multiple charge states of the same precursor peptide sequence(s) are combined. The invention is particularly useful for data-independent acquisition operation of tandem mass spectrometers.

**Background of the Invention**

Tandem mass spectrometry, in particular in combination with liquid chromatography, is a powerful technology to identify and quantify peptides and proteins, particularly in complex samples. The most common method of peptide identification is the data dependent acquisition (DDA) approach, wherein a tandem MS sequentially surveys all peptides that elute from the LC column at a particular retention time and select a few peptide ions (often the few most intense) for fragmentation and mass analysis. Due to the fact that a few peptides are sampled from each MS1 spectrum, each MS1 spectrum being drastically undersampled, the method is inherently stochastic and has low reproducibility. Recent improvements in mass spectrometry have enabled the development of methods of analysing peptide mixtures by data independent acquisition (DIA) where all peptides are systematically fragmented using wide mass isolation windows through which the apparatus cycles.

Data-independent acquisition (DIA) methods aim to expand the benefits of low-throughput targeted proteomics (where particular narrow MS1 ranges are selected for fragmentation and measurement of one or more particular MS2 peaks) to proteome-wide analyses. These methods rely on the use of several broadband isolation windows that select and fragment all peptide ions within a cycle. The resulting datasets can be analysed in an untargeted manner to quantify virtually all peptides present in a sample. Currently, isolation windows differ in that they: (i) exhibit different widths, (ii) are acquired either sequentially or in a non-consecutive order, and (iii) are either juxtaposed or overlapped.

While these strategies have enabled advances in the reproducibility of identifications across samples, thereby enabling confident identification of many more peptides and proteins, the sensitivity and reproducibility of these methods is still well below those that can be achieved with targeted proteomics. Therefore, there is still a need for improvements in methods for performing analysis of samples by tandem mass spectrometry, and in particular in such methods that rely on collections of broad isolation windows.

**Summary of the Invention**

In general terms, the present invention provides new methods of operating a mass spectrometer for analysis of peptide / protein samples, and a mass spectrometer configured to implement the methods.

In one aspect, therefore, the invention provides a method of analysing a sample using a tandem mass spectrometer, the method comprising executing a data acquisition cycle comprising: (i) isolating precursor ions in a mass-to-charge (m/z) isolation window, (ii) fragmenting the precursor ions to generate fragment ions, and (iii) mass analysing the fragment ions, wherein a) the step of isolating precursor ions in an isolation window comprises pooling precursor ions from N>=2 mass-to-charge isolation sub-windows, and b) the boundaries of a first isolation sub-window are defined as $m/z=[x^1_A, x^1_B]$ and the boundaries of the second and any subsequent isolation sub-window are defined as $m/z=$

$$[x^n_{A=}\frac{(z1 \vee x^{n-1}_A - z1) + z2}{z2\vee} \quad , \quad x^n_{B=}\frac{(z1 \vee x^{n-1}_B - z1) + z2}{z2\vee} \quad]$$ where z1 is a first charge state and z2 is a second, different charge state, and $n=\{2,...,N\}$.

In a second aspect, the invention provides a mass spectrometer comprising: an ionisation source for generating ions from a sample; a mass selector for isolating precursor ions in a mass-to-charge (m/z) isolation window; an ion trap for storing precursor ions selected by the mass selector; a dissociation apparatus for fragmenting the precursor ions to generate fragment ions; a mass analyser for analysing the fragment ions; and a controller comprising a processor programmed to: (i) control the mass selector to isolate precursor ions in an isolation window comprising N>=2 mass-to-charge isolation sub-windows and to store those precursor ions in the ion trap, wherein the boundaries of a first isolation sub-window are defined as $m/z=[x^1_A, x^1_B]$ and the boundaries of the second and any subsequent isolation sub-window are defined as $$m/z= [x^n_{A=}\frac{(z1 \vee x^{n-1}_A - z1) + z2}{z2\vee} \quad , \quad x^n_{B=}\frac{(z1 \vee x^{n-1}_B - z1) + z2}{z2\vee} \quad]$$ where z1 is a first charge state and z2 is a second, different charge state, and $n=\{2,...,N\}$; and (ii) control the ion trap to release the precursor ions in the ion trap for fragmentation and analysis.

In a third aspect there is provided a controller for a tandem mass spectrometer, the controller comprising a processor programmed to: (i) control a mass selector of the mass spectrometer to isolate precursor ions in an isolation window comprising N>=2 mass-to-charge isolation sub-windows and to store those precursor ions in an ion trap of the mass spectrometer, wherein the boundaries of a first isolation sub-window are defined as $m/z=[x^1{}_A, x^1{}_B]$ and the boundaries of the second and any subsequent isolation sub-window are defined as $m/z= [x^n{}_{A=\frac{(z1\lor x_A^{n-1}-z1)+z2}{z2\lor}}, x^n{}_{B=\frac{(z1\lor x_B^{n-1}-z1)+z2}{z2\lor}}]$ where z1 is a first charge state and z2 is a second, different charge state, and n={2,...,N}; and (ii) control the ion trap to release the precursor ions in the ion trap for fragmentation and analysis.

Because isolation windows comprise sub-windows that are separated on the mass-to-charge range and that are designed to capture precursor ions that correspond to different charge states of at least one group of precursor analytes, the signal obtained for each isolation window combines information for those precursor analytes that is both reinforcing (by including signals from fragments that are common between different charge states of a particular precursor analyte) and complementary (by including signals from fragments that are unique to certain charge states of the precursor analyte), thereby increasing the signal to noise ratio and confidence of identification of precursor analytes. Further, since the sub-windows are separated in the mass-to-charge range, each isolation window probes non-contiguous regions of a mass-to-charge range and, thus, there is a lower dependency between the number of precursors identified and the time along a separation gradient (retention time) in methods where a collection of isolation windows are repeatedly cycled through in repeated data acquisition cycles covering the m/z range of interest.

In embodiments of any aspect, z1 is a first charge state, and z2 is a second, lower charge state. In other embodiments of any aspect, z1 is a first charge state, and z2 is a second, higher charge state.

In embodiments of any aspect, z1 and z2 are consecutive charges. In other embodiments, z1 and z2 are non-consecutive charges.

In embodiments of the method of the first aspect, isolating precursor ions in an isolation window further comprises pooling precursor ions from M>=1 additional mass-to-charge isolation sub-windows, wherein the boundaries of the first additional mass-to-charge isolation sub-window are defined as $m/z= [x^{m=1}{}_{A=\frac{(z1\lor x_A^1-z1)+z3}{z3\lor}}, x^{m=1}{}_{B=\frac{(z1\lor x_B^{n-1}-z1)+z3}{z3\lor}}]$, and the boundaries of any subsequent additional mass-to-charge isolation sub-window are defined as $m/z= [x^{m=2...M}{}_{A=\frac{(z1\lor x_A^{m-1}-z1)+z3}{z3\lor}}, x^{m=2...M}{}_{B=\frac{(z1\lor x_B^{m-1}-z1)+z3}{z3\lor}}]$, where z3 is a third, different charge state, and m={1,...,M}. In such embodiments, an isolation window pools precursor ions that correspond to three different charge states of at least one group of precursor analytes.

In embodiments, isolating precursor ions in an isolation window comprises pooling precursor ions from N=2 mass-to-charge isolation sub-windows and M=1 additional mass-to-charge isolation sub-window, wherein:

the boundaries of the first isolation sub-window are defined as $m/z=[x^1{}_A, x^1{}_B]$,

the boundaries of the second isolation sub-window are defined as $m/z= [x^n{}_{A=\frac{(z1\lor x_A^1-z1)+z2}{z2\lor}}, x^n{}_{B=\frac{(z1\lor x_B^1-z1)+z2}{z2\lor}}]$, and

the boundaries of the additional mass-to-charge isolation sub-window are defined as $m/z= [x^{m=1}{}_{A=\frac{(z1\lor x_A^1-z1)+z3}{z3\lor}}, x^{m=1}{}_{B=\frac{(z1\lor x_B^{n-1}-z1)+z3}{z3\lor}}]$, where z1 is a first charge state, z2 is a second, different charge state, and z3 is a third, different charge state.

In embodiments of the second or third aspect. the controller processor is programmed to control the mass selector to isolate precursor ions in an isolation window comprising N>=2 mass-to-charge isolation sub-windows and to store those precursor ions in the ion trap, further comprising the controller processor being programmed to control the mass selector to isolate precursor ions in M>=1 additional mass-to-charge isolation sub-windows, and to store those additional precursor ions in the ion trap, wherein the boundaries of the first additional mass-to-charge isolation sub-window are defined as $m/z= [x^{m=1}_{A=\frac{(z1 \lor x^1_A - z1) + z3}{z3 \lor}} , x^{m=1}_{B=\frac{(z1 \lor x^{n-1}_B - z1) + z3}{z3 \lor}}]$, and the boundaries of any subsequent additional mass-to-charge isolation sub-windows are defined as

$m/z= [x^{m=2...M}_{A=\frac{(z1 \lor x^{m-1}_A - z1) + z3}{z3 \lor}} , x^{m=2...M}_{B=\frac{(z1 \lor x^{m-1}_B - z1) + z3}{z3 \lor}}]$, where z3 is a third, different charge state, and $m=\{1,...,M\}$.

In embodiments of the second or third aspect, the controller processor is programmed to control the mass selector to isolate precursor ions in an isolation window comprising N=2 mass-to-charge isolation sub-windows and M=1 additional mass-to-charge isolation sub-window and to store those precursor in the ion trap, wherein:

the boundaries of the first isolation sub-window are defined as $m/z=[x^1_A, x^1_B]$,

the boundaries of the second isolation sub-window are defined as $m/z= [x^n_{A=\frac{(z1 \lor x^1_A - z1) + z2}{z2 \lor}}$ , $x^n_{B=\frac{(z1 \lor x^1_B - z1) + z2}{z2 \lor}}]$, and

the boundaries of the additional mass-to-charge isolation sub-window are defined as $m/z= [x^{m=1}_{A=\frac{(z1 \lor x^1_A - z1) + z3}{z3 \lor}} , x^{m=1}_{B=\frac{(z1 \lor x^{n-1}_B - z1) + z3}{z3 \lor}}]$, where z1 is a first charge state, z2 is a second, different charge state, and z3 is a third, different charge state.

In embodiments of any aspect, z1, z2 and z3 are consecutive charges. In other embodiments, z1, z2 and z3 are non-consecutive charges.

In embodiments of the method of the first aspect, executing a data acquisition cycle comprises repeating steps (i) to (iii) with one or more further isolation window. In some such embodiments, a further isolation window has at least one of $x^1_A$ and/or $x^1_B$ which is a different value from that chosen for the first / preceding isolation window(s) in the data acquisition cycle. In some embodiments, the one or more further isolation windows comprises isolation sub-windows that partially overlap with the sub-windows of at least one of the other isolation windows.

In embodiments of the method of the first aspect, executing a data acquisition cycle comprises repeating steps (i) to (iii) with a plurality of isolation windows, wherein the plurality of isolation windows collectively span a mass-to-charge range of interest. In some such embodiments, the mass-to-charge range of interest corresponds to the mass-to-charge range of the mass spectrometer.

In embodiments of the method of the first aspect, repeating steps (i) to (iii) with at least one further isolation window comprises repeating steps (i) to (iii) with at least one modified parameter selected from $|x^1_A - x^1_B|$, z1, z2, N and z3 and M, if present.

In embodiments of the method of the first aspect, the method comprises repeating the data acquisition cycle. In some embodiments, the method comprises introducing the sample in the mass spectrometer after chromatographic separation of the sample, and repeating the data acquisition cycle comprises repeating the data acquisition cycle at different retention times. In embodiments, repeating the data acquisition cycle at different retention times comprises continuously repeating the data acquisition cycle during elution of the sample. In embodiments, repeating the data acquisition cycle comprises repeating the data acquisition cycle with at least one modified parameter selected from, $x^1_A$ for one or more windows in the data acquisition cycle, $|x^1_A - x^1_B|$, z1, z2, N and z3 and M, if present.

In embodiments of the method of the first aspect, repeating the data acquisition cycle comprises repeating the data acquisition cycle during elution of the sample, wherein at least one data acquisition cycle uses a different value of $|x^1_A - x^1_B|$ from at least one other data acquisition cycle. For example, different widths of isolation subwindows may be used as a function of retention time, such as to use narrow windows at retention times where more analytes enter the mass spectrometer than at retention times where fewer analytes enter the mass spectrometer.

In embodiments of the method of the first aspect, repeating the data acquisition cycle comprises repeating the data acquisition cycle during elution of the sample, where at least one data acquisition cycle uses a different value of $x^1_A$ for one or more windows (or even all windows) in the data acquisition cycle compared to at least one other data acquisition cycle. For example, different group of subwindows may be used towards the beginning and towards the end of the sample separation process (i.e. retention time). One such example is when the location of the subwindows are varied to isolate progressively heavier analytes during the sample separation process (e.g. at the beginning of the sample separation process two subwindows at the low end and towards middle of the m/z range may be used whereas towards the end of the sample separation process two subwindows towards the middle and higher end of the m/range may be used, possibly with a transition comprising the use of three subwindows towards the middle of the sample separation process).

In embodiments of the method of the first aspect, repeating the data acquisition cycle comprises repeating the data acquisition cycle during elution of the sample, wherein at least one data acquisition cycle uses a different combination of values of z1, z2 and z3 (if present) from at least one other data acquisition cycle.

In embodiments of the method of the first aspect, repeating the data acquisition cycle comprises repeating the data acquisition cycle during elution of the sample, wherein at least one data acquisition cycle uses a different value of N and/or M (if present) from at least one other data acquisition cycle. For example, more subwindows could be used in regions of the sample separation gradient where fewer analytes (or analytes in lower amounts) are expected to elute, in order to increase the sensitivity of the method.

In embodiments of the second aspect, the mass selector is a quadrupole. In embodiments, the mass selector is adapted to be able to co-isolate precursor ions in multiple mass-to-charge ranges. In embodiments, the ion trap is a C-trap or an ion-routing multipole. In embodiments, the ion trap is adapted to be able to receive and store precursor ions Pi from more than one separate isolation window (i.e. from multiple non-contiguous mass-to-charge ranges). In embodiments, the mass analyser is a high resolution mass analyser, such as an orbitrap or or a time-of-flight mass analyser.

In embodiments of the second or third aspect, the controller processor is further programmed to repeat steps (i) and (ii) with one or more further isolation windows together forming a data acquisition cycle. In some such embodiments, a further isolation window has at least one of $x^1_A$ and/or $x^1_B$ which is a different value from that chosen for the first / preceding isolation window(s) in the data acquisition cycle. In some embodiments, the one or more further isolation windows comprises isolation sub-windows that partially overlap with the sub-windows of at least one of the other isolation windows.

In embodiments of the second or third aspect, the controller processor is further programmed to repeat steps (i) and (ii) with one or more further isolation windows together forming a data acquisition cycle, wherein the plurality of isolation windows in a data acquisition cycle collectively span a mass-to-charge range of interest. In some such embodiments, the mass-to-charge range of interest corresponds to the mass-to-charge range of the mass spectrometer.

In embodiments of the second or third aspect, the controller processor being programmed to repeat steps (i) and (ii) with one or more further isolation windows together forming a data acquisition cycle comprises the controller processor being programmed to repeat steps (i) and (ii) with at least one modified parameter selected from $|x^1_A - x^1_B|$, $z1, z2, N$ and $z3$ and $M$, if present.

In embodiments of the second or third aspect, the controller processor is further programmed to repeat the data acquisition cycle. In some embodiments, the mass spectrometer is configured to receive a sample from a chromatographic separation device, and the controller processor being programmed to repeat the data acquisition cycle comprises the controller processor being programmed to repeat the data acquisition cycle at different retention times. In embodiments, the controller processor being programmed to repeat the data acquisition cycle at different retention times comprises the controller processor being programmed to continuously repeat the data acquisition cycle during elution of the sample. In embodiments, the controller processor being programmed to continuously repeat the data acquisition cycle comprises the controller processor being programmed to repeat the data acquisition cycle with at least one modified parameter selected from, $x^1_A$ for one or more windows in the data acquisition cycle, $|x^1_A - x^1_B|$, $z1, z2, N$ and $z3$ and M, if present.

In embodiments of the second or third aspect, the controller processor being programmed to repeat the data acquisition cycle comprises the controller processor being programmed to repeat the data acquisition cycle during elution of the sample, wherein at least one data acquisition cycle uses a different value of $|x^1_A - x^1_B|$ from at least one other data acquisition cycle. For example, different widths of isolation subwindows may be used as a function of retention time, such as to use narrow windows at retention times where more analytes enter the mass spectrometer than at retention times where fewer analytes enter the mass spectrometer.

In embodiments of the second or third aspect, the controller processor being programmed to repeat the data acquisition cycle comprises the controller processor being programmed to repeat the data acquisition cycle during elution of the sample, where at least one data acquisition cycle uses a different value of $x^1A$ for one or more windows (or even all windows) in the data acquisition cycle compared to at least one other data acquisition cycle. For example, different group of subwindows may be used towards the beginning and towards the end of the sample separation process (i.e. retention time). One such example is when the location of the subwindows are varied to isolate progressively heavier analytes during the sample separation process (e.g. at the beginning of the sample separation process two subwindows at the low end and towards middle of the m/z range may be used whereas towards the end of the sample separation process two subwindows towards the middle and higher end of the m/range may be used, possibly with a transition comprising the use of three subwindows towards the middle of the sample separation process).

In embodiments of the second or third aspect, the controller processor being programmed to repeat the data acquisition cycle comprises the controller processor being programmed to repeat the data acquisition cycle during elution of the sample, wherein at least one data acquisition cycle uses a different combination of values of z1, z2 and z3 (if present) from at least one other data acquisition cycle.

In embodiments of the second or third aspect, the controller processor being programmed to repeat the data acquisition cycle comprises repeating the data acquisition cycle during elution of the sample, wherein at least one data acquisition cycle uses a different value of N and/or M (if present) from at least one other data acquisition cycle. For example, more subwindows could be used in regions of the sample separation gradient where fewer analytes (or analytes in lower amounts) are expected to elute, in order to increase the sensitivity of the method.

In embodiments of any aspect, N is between 2 and 6. In some embodiments, N is 2, 3 or 4.

In embodiments of any aspect, N is 3 and the boundaries of the three isolation sub-windows are defined as:

sub-window 1: $m/z=[x^1_A, x^1_B]$

sub-window 2: $m/z= [x^2_{A=}\dfrac{(z1*x^1_A-z1)+z2}{z2}, x^2_{B=}\dfrac{(z1*x^1_B-z1)+z2}{z2}]$

sub-window 3: $m/z= [x^3_{A=}\dfrac{(z1*x^2_A-z1)+z2}{z2}, x^2_{B=}\dfrac{(z1*x^2_B-z1)+z2}{z2}]$

In embodiments of any aspect, M is between 1 and 5. In embodiments, M is 1, 2 or 3.

In embodiments of any aspect, z1 and z2 are each chosen to be between +1 and +12. In embodiments, z1 and z2 are individually chosen from the group consisting of: +1, +2, +3, +4, +5, +6, +7, +8, +9, +10, +11 and +12. In embodiments, z1 and z2 are individually chosen from the group consisting of: +1, +2, +3, +4, +5, +6, +8, +9 and +12.

In embodiments of any aspect, z1 and z2 are +2 and +3, respectively. In some such embodiments, the sample comprises peptides and the precursor ions are peptide ions.

In embodiments of any aspect, z1 and z2 are +8 and +9, respectively. In some such embodiments, the sample comprises proteins and the precursor ions are ionised proteins. In some such embodiments, the proteins are intact or native proteins.

In embodimentsof any aspect, z1 and z2 are +8 and +12, respectively. In some such embodiments, the sample comprises proteins and the precursor ions are ionised proteins. In some such embodiments, the proteins are intact or native proteins.

In embodiments of any aspect, z1 and z2 are each chosen to be between -1 and -12. In embodiments, z1 and z2 are individually chosen from the group consisting of: -1, -2, -3, -4, -5, -6, -7, -8, -9, -10, -11 and -12. In embodiments, z1 and z2 are chosen from the group consisting of: -1, -2, -3, -4, -5, -9, -8, -9 and -12.

In embodiments of any aspect, z1 and z3 are -2 and -3, respectively. In some such embodiments, the sample comprises peptides and the precursor ions are peptide ions.

In embodiments of any aspect, z1 and z2 are -8 and -9, respectively. In some such embodiments, the sample comprises proteins and the precursor ions are ionised proteins. In some such embodiments, the proteins are intact or native proteins.

In embodiments of any aspect, z1 and z2 are -8 and -12, respectively. In some such embodiments, the sample comprises proteins and the precursor ions are ionised proteins. In some such embodiments, the proteins are intact or native proteins.

In embodiments of any aspect, the boundaries of the first isolation sub-window are defined as $m/z=[x^1_A, x^1_B]$ wherein $| x^1_A - x^1_B | = w$, and:

    (a) $0 < w \leq 20$ Da, or
    (b) $0 < w \leq 10$ Da, or
    (c) w is 7, 8, 9, 10 or 11 Da, or
    (d) w is 1, 2, 3, 4 or 5 Da.

In embodiments of any aspect, the boundaries of the first isolation sub-window are defined as $m/z=[x^1_A, x^1_B]$ wherein $| x^1_A - x^1_B | = w$, wherein $0 < w \leq 20$ Da or wherein w is between 1 and 20 Da.

In embodiments of any aspect, the boundaries of the first isolation sub-window are defined as $m/z=[x^1_A, x^1_B]$ wherein $| x^1_A - x^1_B | = w$, wherein $0 < w \leq 10$ Da or wherein w is between 1 and 10 Da. In some such embodiments, z1 is a first charge state and z2 is a second, lower charge state.

In embodiments of any aspect, the boundaries of the first isolation sub-window are defined as $m/z=[x^1_A, x^1_B]$ wherein $| x^1_A - x^1_B | = w$, wherein w is 1, 2, 3, 4 or 5 Da. In some such embodiments, z1 is a first charge state and z2 is a second, lower charge state.

In embodiments of any aspect, the boundaries of the first isolation sub-window are defined as $m/z=[x^1_A, x^1_B]$ wherein $| x^1_A - x^1_B | = w$, wherein w is 7, 8, 9, 10 or 11 Da. In some such embodiments, z1 is a first charge state and z2 is a second, higher charge state.]

It will be appreciated that any features of one aspect or embodiment of the invention may be combined with any combination of features in any other aspect or embodiment of the invention, unless otherwise stated, and such combinations are envisaged and are intended to be directly and unambiguously disclosed herein, and to fall within the scope of the present invention.

**Brief Description of the Drawings**

The invention is further illustrated by way of the accompanying drawings in which:

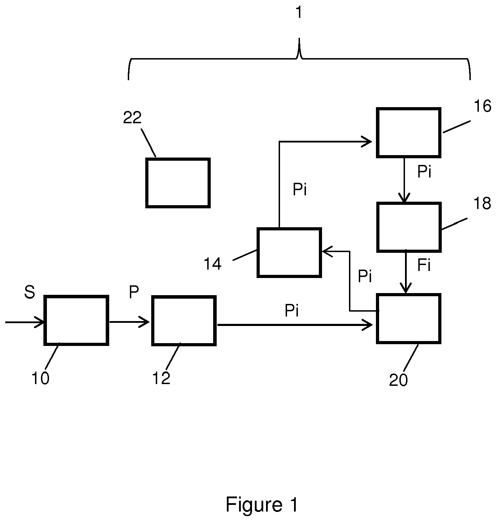

**Figure 1** shows a schematic representation of a mass spectrometer according to embodiments of the invention;

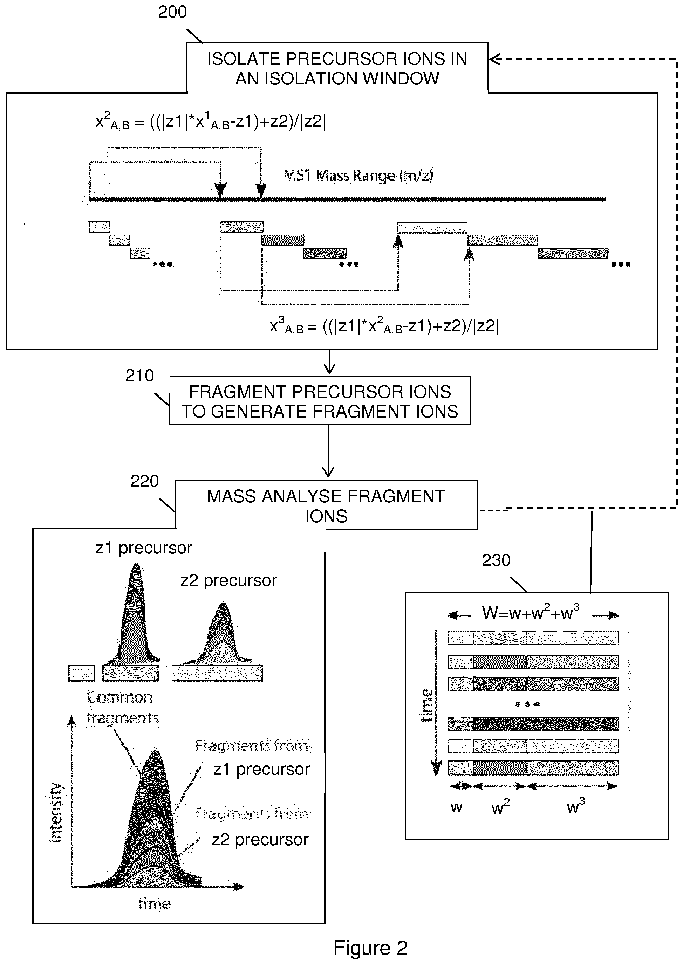

**Figure 2** shows a schematic representation of a method of analysing a sample using a tandem mass spectrometer, according to the invention;

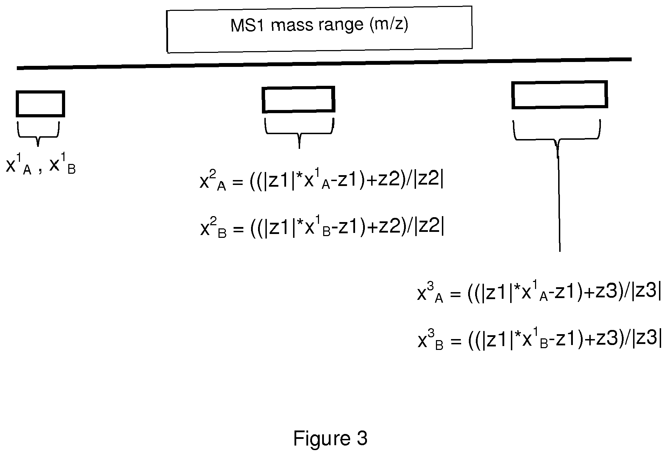

**Figure 3** shows a schematic representation of an embodiment of the method according to the invention, in which three different charges are used;

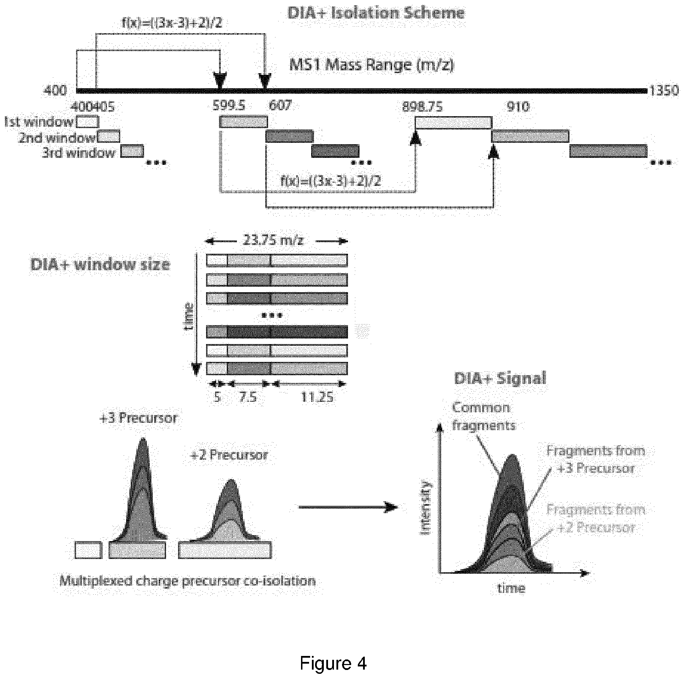

**Figure 4** shows schematically an experimental set-up according to an example of use of the invention;

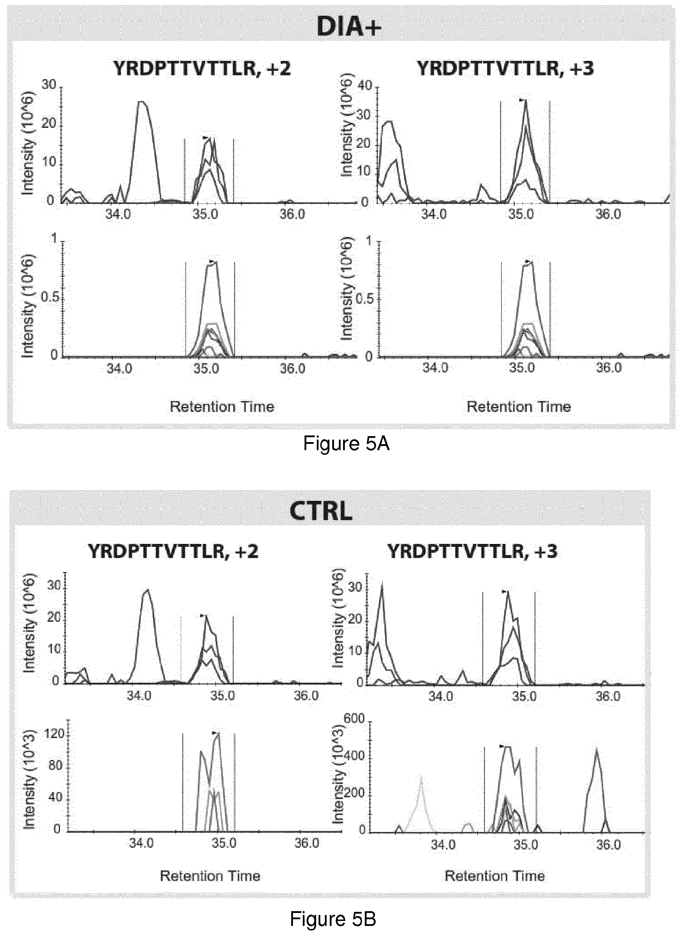

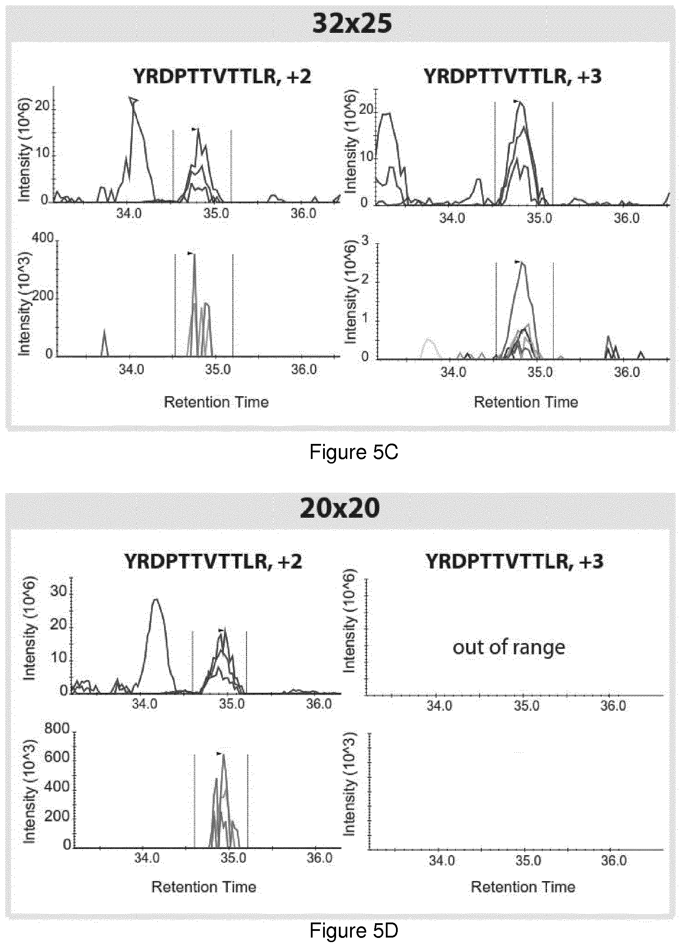

**Figures 5 A to D** show the extracted ion chromatograms for an exemplary peptide (in particular, peptide YRDPTTVTTLR), obtained with the method of the invention (Figure 5A), or with three control methods (Figures 5B, C, D). This data shows that the methods according to the invention result in an increased signal to noise ratio (i.e. some peptides can be identified using the inventive method which could not be confidently identified using control methods, because the combination of fragments from the +2 and +3 precursors of these peptides provide reinforcing (common fragments) and complementary (unique fragments) information in the MS2 spectra);

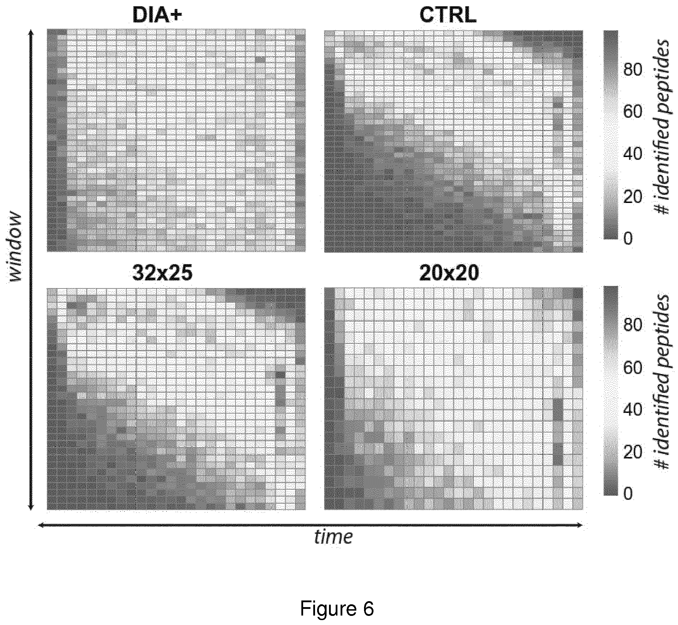

**Figure 6** shows heatmaps of the number of peptides identified in each isolation window in each cycle (i.e. across the retention time scale), obtained in an experiment according to the method of the invention or with three control methods. This shows that using the method of the invention, the number of peptides identified is spread throughout the isolation windows (rows) and data acquisition cycles (columns); by contrast, using the other methods, the identifications are concentrated substantially over the diagonal of the windows x cycle matrix (i.e. many peptides are identified in the first few windows at the beginning of the chromatography gradient and in the last few windows at the end of the chromatography gradient, with a continuous gradient between the two). By contrast, using the methods of the prior art, identifications are concentrated in a few isolation windows in each cycle, thereby: (a) under utilising most of the isolation windows (in which few or no peptides are identified), and (b) reducing the sensitivity in the 'crowded' windows. This data demonstrates that the methods according to the invention enable to spread the identification rate across windows of the data acquisition cycles;

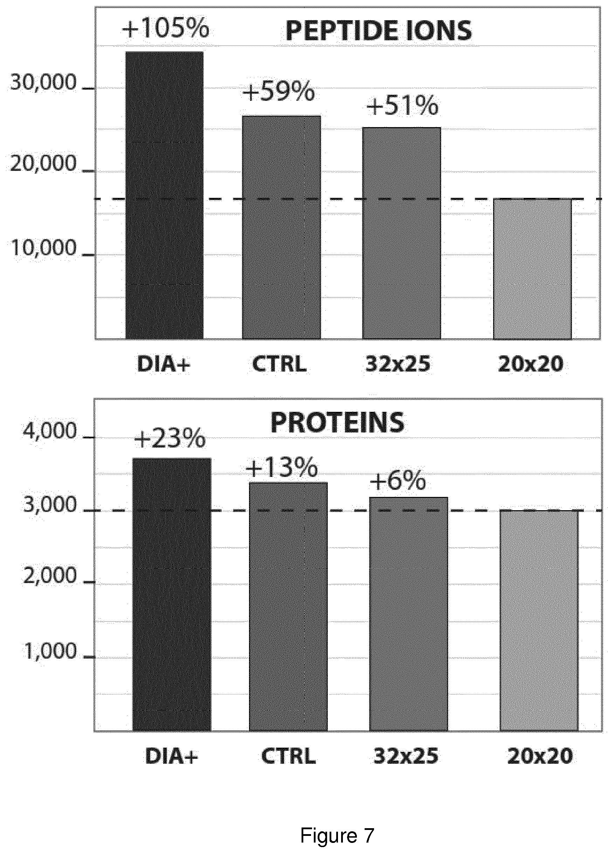

**Figure 7** shows the number of peptides (top) and proteins (bottom) that were confidently identified through an experiment according to a method of the invention or through three control methods, together with the percentage increase compared to the worse performing method (20x20). This shows that using the method of the invention it is possible to identify over twice as many peptides (and over 20% more proteins) compared to using the worse performing control method (20x20), and about 15% more peptides (about 10% more proteins) than the closest matched control method (CTRL), which uses the same number of windows with the same width over the same mass-to-charge range. This data shows that the methods according to the invention have increased sensitivity (i.e. allow to identify an increased number of peptides and proteins) compared to control methods;

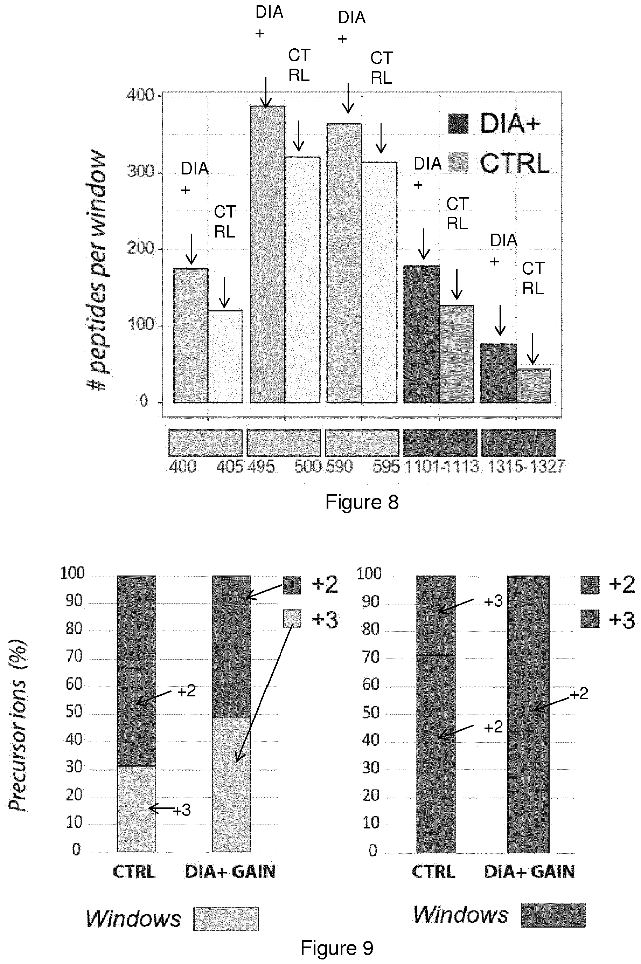

**Figure 8** shows the number of peptides identified per sub-window across all cycles for five sub-windows that are common between an experiment with a control method (CTRL) and the method according to the invention (DIA+). The data shows that even in sub-windows that are common between the two methods, the method according to the invention allows to identify more peptides than the control method. Without wishing to be bound by theory, this is thought to be because the signals from those windows (respectively the signals from +3 ions in the first three sub-windows and from +2 ions in the last two sub-windows) in the inventive method were supplemented with informative signal from the paired window (the third window that lies in between the low m/z windows and the high m/z windows on Figure 4) designed to capture the other charge state (the signals from the +3 ions corresponding to the +2 ions in the last two sub-windows, and the signals from the +2 ions corresponding to the +3 ions in the first three sub-windows) of the peptides that were captured in these windows, thereby enabling more confident identification of the peptides in those particular windows; and

**Figure 9** shows that those peptides that were not identified in the control method in the windows shown on Figure 8, but that were identified in the method according to the invention in those windows are enriched for peptides with the expected charge state for identification as a result of complementary information from corresponding peptides in a different charge state that are co-isolated in a different sub-window, compared to the charge states of the peptides that were identified in the control windows.

**Detailed Description of the Invention**

All references cited herein are incorporated by reference in their entirety. Unless otherwise defined, all technical and scientific terms used herein have the same meaning as commonly understood by one of ordinary skill in the art to which this invention belongs.

Unless otherwise indicated, the practice of the present invention employs conventional techniques in chemistry and chemical methods, biochemistry, data analysis and instrument control, which are within the capabilities of a person of ordinary skill in the art. Such techniques are also described in the literature cited herein, each of which is herein incorporated by reference.

The inventors have discovered that when performing tandem mass spectrometry, by pooling precursor ions that originate from the same molecule but have different charge states, it is possible to obtain a better signal to noise ratio for analysis (identification and optionally quantification) of the precursor ion, and hence identify a larger amount of molecules in a complex sample.

Figure 1 shows a schematic representation of a mass spectrometer according to embodiments of the invention. The mass spectrometer 1 shown on Figure 1 comprises an ionisation source 12 for generating ions from a sample. In the embodiment shown on Figure 1, the mass spectrometer 1 is connected to a sample separation device 10, such as a chromatographic separation device (for example a liquid chromatography (LC) column). In this embodiment, a sample S is introduced in the sample separation device 10, and precursors P that are present in the sample gradually come out of the sample separation device 10 and into the mass spectrometer over time. The time at which a particular precursor P exits the sample separation device is typically called 'retention time' or 'elution time' in the case of liquid chromatography.

In embodiments, the sample is derived from proteins or peptides mixtures, and the precursors P are peptides. When the sample is derived from a protein mixture, it is subject to digestion with an enzyme (for example, trypsin) in order to obtain a peptide mixture. Further, when the sample is derived from a protein sample, it may comprise a peptide mixture obtained using any suitable hydrolytic enzyme known in the art. In embodiments, the sample may comprise intact proteins (see e.g. Catherman *et al.,* 2013; Skinner *et al.* 2018; Fornelli *et al.,* 2018). Of course, it will be appreciated that the sample to be analysed may not in all embodiments comprise or consist of proteins and/or peptides; other complex molecules and complex sample mixtures may equally be used in samples and analysed in accordance with the invention. In embodiments, the sample may comprise any natural or synthetic linear, polymeric, or cyclic molecules, that are amenable to mass spectrometric analysis, and combinations thereof.

Precursors P are then ionised in an ionisation source 12, thereby producing precursor ions Pi. The precursor ions Pi are fed into a first mass analyser 20 to acquire a precursor ion spectrum (also referred to as MS1 spectrum). The precursor ions Pi are then fed into a mass selector 14, which is capable of isolating precursor ions in a defined mass-to-charge (m/z) isolation window. In embodiments, the mass selector 14 may be a quadrupole. In embodiments, the mass selector 14 may be able to co-isolate precursor ions in multiple mass-to-charge ranges. The precursor ions Pi that are selected by the mass selector 14 are stored in an ion trap 16, until the pooled precursor ions in the ion trap 16 are ready for fragmentation and mass analysis. At this point, the precursor ions Pi in the ion trap 16 are fed through a dissociation apparatus 18 for fragmenting the precursor ions to generate fragment ions Fi. Dissociation apparatus may include apparatus that relies on collision-induced dissociation, electron capture dissociation, electron transfer dissociation, amongst others. Dissociation apparatus are known in the art and will not be discussed further herein.

After fragmentation, the fragment ions Fi are introduced into a mass analyser 20, which detects the mass-to-charge ratio of the fragment ions Fi (also referred to as an MS2 spectrum). Preferably, the mass analyser 20 is a high resolution mass analyser, such as an orbitrap or a time-of-flight mass analyser. In the embodiment shown on Figure 1, the same mass analyser 20 generates the MS1 and MS2 spectra (i.e. a single mass analyser is operated in MS1 mode and in MS2 mode). In other embodiments, two separate mass analysers may be provided.

According to the invention, the ion trap 16 is adapted to be able to receive and store precursor ions Pi from more than one separate isolation window (i.e. from multiple non-contiguous mass-to-charge ranges). In other words, the ion trap 16 can receive precursors in a first mass-to-charge isolation window selected by the mass selector 14 and store those precursor ions, then the mass selector 14 may be tuned to select precursor ions Pi from at least one other isolation window and communicate those ions to the ion trap 16 for pooling with the precursor ions Pi already stored in the ion trap 16. In embodiments, the ion trap 16 is a C-trap. In embodiments, the ion trap is an ion-routing multipole. For example, the mass spectrometer may combine a quadrupole, orbitrap and ion trap, such as e.g. the Orbitrap Fusion by Thermo Scientific, or may be a hybrid quadrupole-Orbitrap mass spectrometer such as the Q exactive by Thermo Scientific.

The mass spectrometer 1 further comprises a controller 22 which controls at least the mass selector 14 and the ion trap 16. In embodiments, a single controller 22 is provided to control the ion source 12, mass selector 14, ion trap 16, dissociation apparatus 18 and mass analyser 20.

The controller 22 comprises a processor which is programmed to control the mass selector 14 to isolate precursor ions Pi in an isolation window comprising N>=2 mass-to-charge isolation sub-windows and to store those precursor ions in the ion trap 16. The processor is further programmed to control the ion trap16 to release the precursor ions Pi in the ion trap for fragmentation and analysis.

According to the invention, the sub-windows of an isolation window are designed such that they capture multiple charge states of the same precursor P, as will be further explained below.

Figure 2 shows a schematic representation of a method of analysing a sample using a tandem mass spectrometer, according to the invention.

The method comprising executing a data acquisition cycle comprising: isolating 200 precursor ions in a mass-to-charge (m/z) isolation window, fragmenting 210 the precursor ions to generate fragment ions, and mass analysing 220 the fragment ions. According to the invention, isolating 200 precursor ions Pi in an isolation window comprises pooling precursor ions from N>=2 mass-to-charge isolation sub-windows, wherein the boundaries of a first isolation sub-window are defined as $m/z=[x^1_A, x^1_B]$ and the boundaries of the second and any subsequent isolation sub-window are defined as $m/z=$ $\left[x^n_{A=}\dfrac{(z1 \vee x^{n-1}_A - z1)+z2}{z2\vee}, x^n_{B=}\dfrac{(z1 \vee x^{n-1}_B - z1)+z2}{z2\vee}\right]$ where z1 is a first charge state and z2 is a second, different charge state, and $n=\{2,...,N\}$.

In embodiments, N is between 2 and 6. In convenient embodiments, N is 2, 3 or 4. In one such embodiment, N is 3 and the boundaries of the three isolation sub-windows are defined as:

sub-window 1: $m/z=[x^1_A, x^1_B]$

sub-window 2: $m/z= \left[x^2_{A=}\dfrac{(z1*x^1_A - z1)+z2}{z2}, x^2_{B=}\dfrac{(z1*x^1_B - z1)+z2}{z2}\right]$

sub-window 3: $m/z= \left[x^3_{A=}\dfrac{(z1*x^2_A - z1)+z2}{z2}, x^2_{B=}\dfrac{(z1*x^2_B - z1)+z2}{z2}\right]$

In the embodiment shown on Figure 2, N=3 mass-to-charge isolation sub-windows are used. For example, in such an embodiment if $x^1_A$ is chosen as *400 m/z*, $x^1_B$ is chosen as *405 m/z*, z1 is chosen as +3 and z2 is chosen as +2, then a first isolation window comprises three sub-windows with respective boundaries [400, 405], [599.5, 607] and [898.75, 910]. In embodiments where N=3, as shown in Figure 2, the first and second sub-windows will capture the first and second charge states of a first group of precursors, whereas the second and third sub-windows will capture the first and second charge states of a second group of precursors. Similarly, in embodiments with N=4 or more, the first and second sub-windows will capture the first and second charge states of a first group of precursors, the second and third sub-windows will capture the first and second charge states of a second group of precursors, the third and fourth sub-windows will capture the first and second charge states of a third group of precursors, etc. In the simplest embodiments, N can be two and the isolation window can comprise two sub-windows which each capture a different charge state of the same group of precursors.

As the skilled person would understand, each sub-window may also capture precursor ions which do not belong to a group of precursors for which another charge state is captured in another window. For those precursors, the multiplexing (combination of sub-windows as described) will not result in an increase of the signal to noise ratio. However, this does not detract from the benefit associated with increasing the signal-to-noise ratio for those precursors that have multiple charge states which will be captured in the different sub-windows.

In the embodiment shown on Figure 2, z1 is a first charge state, and z2 is a second, lower charge state (i.e. the second sub-window is higher in the m/z range than the first sub-window). In other embodiments, z1 is a first charge state, and z2 is a second, higher charge state. In such embodiments, a first sub-window $[x^1_A, x^1_B]$ is selected in the m/z range of the spectrometer and second and subsequent sub-windows are located lower in the m/z range of the mass spectrometer.

As used herein, the terms 'higher' or 'lower' when referring to charge states refers to the absolute value of the charge. For example, a +3 precursor has a higher charge state than a +2 precursor. Similarly, a - 3 precursor has a higher charge state than a -2 precursor.

In embodiments, z1 and z2 may be consecutive charges. For example, z1 and z2 may be +2 and +3, respectively. Such embodiments may be particularly useful in analysis of protein / peptide samples, where +2 and +3 precursor ions are expected to be particularly abundant. In other embodiments, z1 and z2 are non-consecutive charges. For example, z1 and z2 may be +2 and +4, respectively.

As the skilled person would understand, the methods of the invention may be used in positive ion mode (where the charges z1, z2 and z3, if present, are positive), or in negative ion mode (where the charges z1, z2 and z3, if present, are negative). Further, the choice of which charges to use may depend on the particular use case, the nature of the sample as well as the purpose of the analysis.

In embodiments, z1 and z2 are chosen from the group consisting of: +1, +2, +3, +4, +5, +6, +7, +8, +9 and +12. In other embodiments, z1 and z2 are chosen from the group consisting of: -1, -2, -3, -4, -5, -6, -7, -8, -9 and -12.

For example, the use of negative charges z1 and z2 may be advantageous to analyse molecules or modified peptides with negatively charged moieties.

In embodiments, the use of high charges may be particularly useful to intact protein analysis (see e.g. Catherman *et al.,* 2013; Skinner *et al.* 2018). In embodiments, z1 and z2 may be +8 and +9, respectively; or +8 and +12, respectively. Such embodiments may be particularly useful for the analysis of samples comprising intact proteins (rather than peptides).

As can be seen in Figure 2, the width of a first isolation sub-window can be expressed as $w = |x^1_A - x^1_B|$. As the skilled person would understand, the width of the further isolation sub-windows is derived from w and will depend on the relative charges z1 and z2. In the example shown on Figure 2, three sub-windows are used with widthds $w^2 = |x^2_A - x^2_B|$ and $w^3 = |x^3_A - x^3_B|$, leading to an isolation window with a total width of $W = w + w^2 + w^3$. In embodiments, the width w of each first isolation sub-window is chosen to be between 1 and 20 m/z units (also expressed in Da as z is unitless), or such that $0 < w \leq 20$ Da. In embodiments, the width w of each first isolation sub-window may be chosen to be between 1 and 10 Da or such that $0 < w \leq 10$ Da. In embodiments, w is 1, 2, 3, 4 or 5 Da. In some such embodiments, z1 is a first charge state and z2 is a second, lower charge state (i.e. each first isolation sub-window is lower in the m/z range and narrower than its associated second isolation sub-window, etc). In other embodiments, w is 7, 8, 9, 10 or 11 Da. In some such embodiments, z1 is a first charge state and z2 is a second, higher charge state (i.e. each first isolation sub-window is higher in the m/z range and wider than its associated second isolation sub-window, etc).

As can be seen schematically on Figure 2, the result of the use of an isolation window as described (also referred to as 'multiplexed isolation window') is that multiple charge states of the same precursor will be selected, pooled and their fragments mass analysed together. As a result, the signal corresponding to fragments that are common to the different charge states will be reinforced, and further signals will be provided corresponding to fragments that are unique to each charge state, thereby providing more information for the identification of the precursor.

As the skilled person would understand, the result of mass analysis of the multiplexed isolation window can be analysed using any mass spectrometry data analysis method known in the art. For example, the data may be analysed using a pipeline such as DIA-Umpire (Tsou *et al.,* 2015), and/or library-based strategies (Gillet *et al.* 2012).

As the skilled person would understand, the number of sub-windows used and their widths interplay to influence the complexity of the data resulting from the method. Indeed, wider sub-windows with a fixed number of sub-windows are expected to capture more precursors resulting in more complex data.

Similarly, a higher number of sub-windows implies a larger total width with a fixed width w of the first sub-window, thereby resulting in an increased complexity.

Further, the narrower the isolation window the less complex the resulting data, and the more likely it is that a larger proportion of the peptides in the isolation window will be confidently identified. However, the width of the windows may be limited by the cycling ability of the mass spectrometer, since it is necessary to re-tune the mass selector between each isolation window and sub-window. If the cycling speed of the mass spectrometer is too slow (i.e. the mass spectrometer is being made to cycle through many settings such that completing a cycle takes a certain amount of time), it is more likely that precursors in the sample may be missed because they were not in a m/z range that was being selected at the time where they entered the mass spectrometer from the sample separation device. Therefore, for a given m/z range to be analysed in each cycle, a balance has to be reached between the smallest total width W of isolation window that can be used (and hence the number of isolation windows used in the cycle) without sacrificing sensitivity due to slow cycling. This balance will depend highly on the particular apparatus that is used, as the cycling speed of commercially available apparatus is ever increasing.

In embodiments, steps 200 to 220 can be repeated 230 for one or more further isolation windows, each being composed of sub-windows as described. A further isolation window has at least one of $x^1_A$ and/or $x^1_B$ which is a different value from that used for the first or any of the preceding isolation window(s) in the data acquisition cycle. In the embodiment shown on Figure 2, the sub-windows that form each isolation window are adjacent and do not overlap. In other embodiments, the one or more further isolation windows comprises isolation sub-windows that partially overlap with the sub-windows of at least one of the other isolation windows. For example, it is possible to define the boundaries $x^1_A$, $x^1_B$ for each isolation window such that those boundaries define region with a slight m/z overlap, forming what can be referred to as a tiling. In embodiments, the regions may overlap by a chosen percentage of their range, such as 10, 20 or 50%.

In embodiments where consecutive isolation sub-windows overlap by 50% of their range, each region of the m/z range may be isolated twice successively.

Throughout this disclosure, repeating steps 200 to 220 with a collection of isolation windows is referred to as a data acquisition cycle. In embodiments, a data acquisition cycle comprises repeating steps 200 to 220 with a plurality of isolation windows, wherein the plurality of isolation windows collectively span a mass-to-charge range of interest. In such embodiments, the data acquisition cycle effectively scans the range of interest. For example, a mass-to-charge ratio of interest may be a user-defined mass-to-charge ratio (which may be discontinuous), in which precursors of interest are expected to be located. This can be defined based on e.g. a list of precursors of interest, or, for example, empirically using prior data. In embodiments, the mass-to-charge range of interest corresponds to the mass-to-charge range of the mass spectrometer. In such embodiments, a data analysis cycle may be designed to scan the entire range of the mass spectrometer, for example, to perform an unbiased discovery of all the precursors that may be present in the sample and that can be identified by mass spectrometry.

In embodiments, as shown on Figure 2, the isolation windows that together make up a collection of isolation windows from a data acquisition cycle may have the same parameters $|x^1_A - x^1_B|$, $z1$, $z2$, $N$ and $z3$ and $M$, if present (see below). In other embodiments, repeating steps 200 to 220 with a collection of isolation windows in a data acquisition cycle may comprise changing at least one parameter during the data acquisition cycle. For example, any of $|x^1_A - x^1_B|$, $z1$, $z2$, $N$ and $z3$ and $M$ (see below), if present, may be varied. In these embodiments, a collection of isolation windows in a data acquisition cycle may have different total widths W, different sub-windows widths w, different combinations of charges, and/or different numbers of sub-windows.

In embodiments, successive isolation windows in a data acquisition cycle may be consecutive in mass-to-charge (i.e. each of the first sub-windows of the successive isolation windows covers progressively higher or lower mass-to-charge ranges), or non-consecutive (for example, each of the first sub-windows of the successive isolation windows may be chosen randomly within a range, such that two successive first sub-windows are not necessarily consecutive in the mass-to-charge range). In embodiments where the successive isolation windows are non-consecutive (i.e. their individual sub-windows are non-consecutive when compared to corresponding sub-windows of the preceding isolation window), the location of the isolation windows may be chosen randomly amongst possible locations, or according to a predetermined scheme.

Figure 3 shows a schematic representation of an embodiment of the method according to the invention, in which three different charges are used. In the embodiment shown on Figure 3, isolating precursor ions in an isolation window further comprises pooling precursor ions from M>=1 additional mass-to-charge isolation sub-windows, in addition to the N mass-to-charge isolation sub-windows described above. The boundaries of the first additional mass-to-charge isolation sub-window are defined as $m/z=\left[x^{m=1}_{A=\frac{(z1\vee x^1_A-z1)+z3}{z3\vee}}, x^{m=1}_{B=\frac{(z1\vee x^{n-1}_B-z1)+z3}{z3\vee}}\right]$, and the boundaries of any subsequent additional mass-to-charge isolation sub-windows are defined as $m/z=\left[x^{m=2...M}_{A=\frac{(z1\vee x^{m-1}_A-z1)+z3}{z3\vee}}, x^{m=2...M}_{B=\frac{(z1\vee x^{m-1}_B-z1)+z3}{z3\vee}}\right]$, where z3 is a third, different charge state, and $m=\{1,...,M\}$.

In the embodiment shown in Figure 3, N=2 and M=1, such that three sub-windows are used wherein:

the boundaries of the first isolation sub-window are defined as $m/z=[x^1_A, x^1_B]$,

the boundaries of the second isolation sub-window are defined as $m/z=\left[x^n_{A=\frac{(z1\vee x^1_A-z1)+z2}{z2\vee}}, x^n_{B=\frac{(z1\vee x^1_B-z1)+z2}{z2\vee}}\right]$, and

the boundaries of the additional mass-to-charge isolation sub-window are defined as $m/z=\left[x^{m=1}_{A=\frac{(z1\vee x^1_A-z1)+z3}{z3\vee}}, x^{m=1}_{B=\frac{(z1\vee x^{n-1}_B-z1)+z3}{z3\vee}}\right]$, where z1 is a first charge state, z2 is a second, different charge state, and z3 is a third, different charge state. In such embodiments, the combined isolation window captures three different charge states of a first group of precursors. As the skilled person would understand, the method of Figure 3 can be equally applied with N>2 and M>1, in which case the multiple charge states of more than one group of precursors may be captured.

In embodiments, M is between 1 and 5, preferably M is 1, 2 or 3.

In embodiments, z1, z2 and z3 may be consecutive charges. In embodiments, z1, z2 and z3 may be non-consecutive charges. Further, any of z1, z2 and z3 may be consecutive or non-consecutive. As the skilled person would understand, any of the features described in relation to Figure 2 apply equally to the method described in relation to Figure 3. For example, the process may be repeated with further isolation windows to form a data acquisition cycle, the isolation windows in a data acquisition cycle may be partially overlapping, a data acquisition cycle may be repeated, etc.

In embodiments of any of the methods of the invention, the method may comprise repeating the data acquisition cycle. For example, as explained above, a sample to be analysed may be gradually introduced in the mass spectrometer used to implement the method via a sample separation device. In such embodiments, the method may comprise repeating the data acquisition cycle at different retention times. In some embodiments, the method may comprise continuously repeating the data acquisition cycle during elution of the sample.

In embodiments, each repetition of the data acquisition cycle may be performed with the same parameters. In other embodiments, repeating the data acquisition cycle comprises repeating the data acquisition cycle with at least one modified parameter. The modified parameter may be selected from $[x^1_A, x^1_B]$ for one or more windows in a collection, $|x^1_A - x^1_B|$, z1, z2, N and z3 and M (if present), or even the strategy used to select consecutive isolation windows. This may be particularly advantageous in any situation where the mass-to-charge ratios of analytes of interest are not distributed equally along the separation time scale (retention time). Indeed, in such cases it may be advantageous to tailor the parameters used in a data acquisition cycle to the particular mass-to-charge distribution that is expected over the range of elution times that the data acquisition cycle covers.

For example, it may be advantageous to use different widths of isolation windows as a function of retention time through a sample separation device. This enables smaller widths to be used at retention times where the eluting sample is complex, thereby increasing the sensitivity of the method, and larger widths to be used at retention times where the eluting sample is less complex (i.e. fewer precursors are expected to elute at the same time), thereby maintaining efficiency and resolution of data acquisition.

As another example, it may be advantageous to use different $[x^1_A, x^1_B]$ in a collection depending on the retention time. This may enable multiple charge states of light precursors to be analysed in the data acquisition cycles at retention times where such precursors are expected to occur (e.g. at the beginning of an LC elution), and multiple charge states of heavier precursors to be analysed in the data acquisition cycles at retention times where such precursors are expected to occur (e.g. later in an LC elution). For example, in the example shown on Figure 2, the first and second sub-windows only could be used at the beginning of the elution, then the second and third sub-windows could be used towards the end of the elution.

As a further example, it may be advantageous to use different combinations of charges z1, z2 and optionally z3 at different retention times. For example, it may be advantageous to use two charges in some regions of the elution gradient (such as e.g. where the signal from pooled precursors with two charged states is expected to be sufficient to identify most peptides of interest) and three charges in other regions (for example, in regions where the additional sensitivity that comes with pooling three charge states would be of interest).

As yet another example, it may be advantageous to combine more sub-windows (e.g. pooling precursor ions from more groups of precursors) in some regions of the elution gradient and fewer sub-windows in other regions. In other words, it may be advantageous to change N and/or M (if present) between data acquisition cycles. For example, it may be beneficial to use fewer sub-windows at retention times where the eluting sample is complex, and more sub-windows at retention times where the eluting sample is less complex.

Without wishing to be bound by theory, it is believed that the methods of the invention will be particularly useful in discovery analysis of complex protein or peptide mixtures and, in particular, in data-independent acquisition methods of performing tandem mass spectrometry.

The invention will now be further illustrated by way of the following non-limiting examples.

## Examples

Unless otherwise indicated, commercially available reagents and standard techniques in biology, chemistry and biochemistry were used.

### *Example 1: analysis of peptide mixtures from digested HeLa cell extracts*

Peptide mixtures derived from digested HeLa cell extracts were injected in triplicates in an Orbitrap Fusion Lumos mass spectrometer (MS1 orbitrap detection Resolution=60K, quadrupole isolation, HCD fragmentation with CE=28%, Max IT=60ms, MS2 Orbitrap detection Resolution=30K) equipped with a chromatographic nano-UPLC system (Proxeon-1000) and a 50-cm C18 column (EASY-Spray; 75μm, PepMap RSLC C18, 2μm particles, 45°C) using 120-min chromatographic gradients (ACN:H2O, 1% FA).

1 μg of HeLa tryptic digest was injected in triplicate and each replicate was analysed according to one of four different methods:

(1) The method of the invention, with parameters as shown on Figure 4 (designated in Figures 5 to 8 as "DIA+"):

- 40 isolation windows together forming a collection that covers the m/z range between 400 and 1350 m/z were used;
- each isolation window comprises a first sub-window that has a width of 5 m/z, where the first sub-window of each successive isolation window is adjacent to (and non-overlapping with) the first sub-window of the preceding isolation window in the data acquisition cycle;
- N=3, M=0 (i.e. only two charge states are used);
- $z_1$=+3, $z_2$=+2;
- leading to a first sub-window width w = 5 Da, a second sub-window width of $w^2$ = 7.5 Da and a third sub-window width $w^3$ = 11.25, with a total isolation window width W = 5+7.5+11.25 = 23.75 Da;

(2) A control method (designated in Figures 5 to 8 as 'CTRL') using isolation windows of the same width as used in accordance with the method of the invention (23.75 m/z) but comprising three consecutive sub-windows of the same sizes as the sub-windows of the method according to the invention (i.e. the mass selector isolates and pools three consecutive mass windows of respectively 5, 7.5 and 11.25 Da), forming a collection of 40 isolation windows per data acquisition cycle, together covering the mass-to-charge range between 400 and 1350 Da;

(3) Two further control methods which correspond to reference methods in the field:

(3a) 32 partially overlapping consecutive windows of 25 Da together covering a m/z range between 400 and 1200 da (as in Gillet *et al.*, 2012) (designated in Figures 5 to 8 as "32x25");
(3b) 20 consecutive non-overlapping windows of 20 Da together covering a m/z range of 500 to 900 Da (designated in Figures 5 to 8 as "20x20").

*Example 2: the methods according to the invention result in an increased signal to noise ratio compared to prior art methods*

Figure 5 shows the extracted ion chromatograms for an exemplary peptide (in particular, peptide YRDPTTVTTLR) which could only be confidently identified in Example 1 using the method of the invention. Individual lines represent the intensity for individual ions (precursors or fragments, as the case may be) as a function of retention time.
In particular, Figure 5A shows the extracted ion chromatograms obtained with the method of the invention, for the +2 and +3 precursor ions as well as the combined MS2 spectrum which could be obtained because both precursor ions were fragmented and analysed together.
Figures 5B, C and D respectively show the extracted ion chromatograms obtained with the three control methods (CTRL, 32x25 and 20x20, respectively). While the +2 and +3 precursor ions were detected in the MS1 spectra, the peptide was not confidently identified using any of these methods.
This data indicates that the methods according to the invention result in an increased signal to noise ratio (i.e. some peptides can be identified using the inventive method which could not be confidently identified using control methods, because the combination of fragments from the +2 and +3 precursors of these peptides provide reinforcing (common fragments) and complementary (unique fragments) information in the MS2 spectra).

*Example 3: the methods according to the invention result in a more advantageous utilisation of the DIA cycle compared to prior art methods*

Figure 6 shows heatmaps of the number of peptides identified in each isolation window in each cycle (i.e. across the retention time scale), in Example 1. The data on Figure 6 shows that using the method of the invention, the number of peptides identified are spread throughout the isolation windows (rows) and data acquisition cycles (columns). By contrast, using the other methods, the identifications are concentrated substantially over the diagonal of the windows x cycle matrix (i.e. many peptides are identified in the first few windows at the beginning of the chromatography gradient and in the last few windows at the end of the chromatography gradient, with a continuous gradient between the two). As a result, using the methods of the prior art, identifications are concentrated in a few isolation windows in each cycle, thereby (a) under utilising most of the isolation windows (in which few or no peptides are identified), and (b) reducing the sensitivity in the 'crowded' windows.
This data demonstrates that the methods according to the invention enable the identification rate to be spread across windows of the data acquisition cycles.

*Example 4: the methods of the invention result in an increase in the number of peptides and proteins confidently identified, compared to prior art methods*

Figure 7 shows the number of peptides (top) and proteins (bottom) that were confidently identified in the experiment of Example 1, together with the percentage increase compared to the worse performing method (20x20). We note that the worse performing method has a narrower mass-to-charge range than the other three and as such fewer identifications are expected to some extent.

The data in Figure 7 shows that using the method of the invention it is possible to identify over twice as many peptides (and over 20% more proteins) as using the worse performing control method (20x20), and about 15% more peptides (about 10% more proteins) than the closest matched control method (CTRL) which uses the same number of windows with the same width over the same mass-to-charge range.

The data on Figure 7 shows that the methods according to the invention have increased sensitivity (i.e. allow the identification of an increased number of peptides and proteins) compared to control methods.

*Example 5: the methods of the invention result in an increase in the number of peptides identified due to charge multiplexing*

Figure 8 shows the number of peptides identified per sub-window across all cycles for five sub-windows that are common between the control method (CTRL) and the method according to the invention (DIA+). The data shows that even in sub-windows that are common between the two methods, the method according to the invention allows the identification of more peptides than the control method. This is because the signals from those windows (respectively the signals from +3 ions in the first three sub-windows and from +2 ions in the last two sub-windows) in the inventive method were supplemented with informative signal from the paired window (the third window that lies in between the low m/z windows and the high m/z windows on Figure 4) designed to capture the other charge state (the signals from the +3 ions corresponding to the +2 ions in the last two sub-windows, and the signals from the +2 ions corresponding to the +3 ions in the first three sub-windows) of the peptides that were captured in these windows, thereby enabling more confident identification of the peptides in those particular windows.

Figure 9 shows that those peptides that were not identified in the control method in the windows shown on Figure 8, but that were identified in the method according to the invention are enriched for peptides with the expected charge state for identification as a result of the complementary information from corresponding peptides in different charge states that are co-isolated in a different sub-window, compared to the charge states of the peptides that were identified in the control windows. In particular, while in the first three windows of Figure 8 (i.e. m/z = 400-405, 495-500 and 590-595) in the control method both +2 and +3 peptides were identified (about 70 and 30%, respectively - see bar labelled "CTRL" on the left hand plot of Figure 9), whereas almost 50% of the peptides that were only identified in the method according to the invention (bar labelled "DIA+GAIN") are +3 peptides. Similarly, while in the last two windows of Figure 8 (i.e. m/z = 1101-1113 and 1315-1327) in the control method both +2 and +3 peptides were identified (about 70 and 30%, respectively - see bar labelled "CTRL" on the right hand plot of Figure 9), almost 100% of the peptides that were only identified in the method according to the invention (bar labelled "DIA+GAIN") are +2 peptides (i.e. the identification gain is enriched in +2 peptides). This is the expected result if the gain in identification in the low m/z sub-windows (left hand plot on Figure 9) is due to the additional information about +3 charged precursors from the corresponding +2 charged precursors that are present in the multiplexed window of the method of the invention but are not present in the window of the control method. Similarly, this is the expected result if the gain in identification in the high m/z sub-windows (right hand plot on Figure 9) is due to the additional information about +2 charged precursors from the corresponding +3 charged precursors that are present in the multiplexed window of the method of the invention but are not present in the window of the control method.

This data indicates that the increased identification seen using the methods of the invention is at least partially due to charge multiplexing which is implemented in the method.

*References*

Catherman AD1, Durbin KR, Ahlf DR, Early BP, Fellers RT, Tran JC, Thomas PM, Kelleher NL. Large-scale top-down proteomics of the human proteome: membrane proteins, mitochondria, and senescence. Mol Cell Proteomics. 2013 Dec;12(12):3465-73. doi: 10.1074/mcp.M113.030114. Epub 2013 Sep 10.

Luca Fornelli, Timothy K. Toby, Luis F. Schachner, Peter F. Doubleday, Kristina Srzentic, Caroline J. DeHart, Neil L. Kelleher. Top-down proteomics: Where we are, where we are going? Journal of Proteomics Volume 175, 20 March 2018, Pages 3-4 https://doi.org/10.1016/j.jprot.2017.02.002 Ludovic C. Gillet, Pedro Navarro, Stephen Tate, Hannes Röst, Nathalie Selevsek, Lukas Reiter, Ron Bonner and Ruedi Aebersold. Targeted Data Extraction of the MS/MS Spectra Generated by Data-independent Acquisition: A New Concept for Consistent and Accurate Proteome Analysis. First Published on January 18, 2012, doi: 10.1074/mcp.O111.016717 June 1, 2012 Molecular & Cellular Proteomics, 11, 0111.016717.

Skinner, O S; Haverland, N A; Fornelli, L; Melani, R D; Vale, Do L H F; Seckler, H S; Doubleday, P F; Schachner, L F; Srzentic, K; Kelleher, N L; Compton, P D Top-down characterization of endogenous protein complexes with native proteomics. Journal Article Nat. Chem. Biol., 2017. doi: 10.1038/nchembio.2515

Chih-Chiang Tsou, Dmitry Avtonomov, Brett Larsen, Monika Tucholska, Hyungwon Choi, Anne-Claude Gingras & Alexey I Nesvizhskii. DIA-Umpire: comprehensive computational framework for data-independent acquisition proteomics. Nature Methods volume 12, pages 258-264 (2015) doi: 10.1038/nmeth.3255

**Claims**

1. A method of analysing a sample using a tandem mass spectrometer, the method comprising executing a data acquisition cycle comprising:

   (i) isolating precursor ions in a mass-to-charge (m/z) isolation window,
   (ii) fragmenting the precursor ions to generate fragment ions, and
   (iii) mass analysing the fragment ions,

   wherein isolating precursor ions in an isolation window comprises pooling precursor ions from $N>=2$ mass-to-charge isolation sub-windows,
   wherein the boundaries of a first isolation sub-window are defined as $m/z=[x^1_A, x^1_B]$ and the boundaries of the second and any subsequent isolation sub-window are defined $m/z= [x^n_{A=\frac{(z1\vee x_A^{n-1}-z1)+z2}{z2\vee}}$ ,

   $x^n_{B=\frac{(z1\vee x_B^{n-1}-z1)+z2}{z2\vee}}]$ where z1 is a first charge state and z2 is a second, different charge state, and $n=\{2,...,N\}$.

2. The method of claim 1, wherein isolating precursor ions in an isolation window further comprises pooling precursor ions from $M>=1$ additional mass-to-charge isolation sub-windows, wherein the boundaries of the first additional mass-to-charge isolation sub-window are defined as $m/z= [x^{m=1}_{A=\frac{(z1\vee x_A^1-z1)+z3}{z3\vee}}$ , $x^{m=1}_{B=\frac{(z1\vee x_B^{n-1}-z1)+z3}{z3\vee}}]$,
   and the boundaries of any subsequent additional mass-to-charge isolation sub-window are defined as

   $m/z= [x^{m=2...M}_{A=\frac{(z1\vee x_A^{m-1}-z1)+z3}{z3\vee}}$ , $x^{m=2...M}_{B=\frac{(z1\vee x_B^{m-1}-z1)+z3}{z3\vee}}]$, where z3 is a third, different charge state, and $m=\{1,...,M\}$.

3. The method of any preceding claim, wherein executing a data acquisition cycle comprises repeating steps (i) to (iii) with one or more further isolation window.

4. The method of claim 3, wherein the one or more further isolation windows comprises isolation sub-windows that partially overlap with the sub-windows of at least one of the other isolation windows.

5. The method of any preceding claim, wherein executing a data acquisition cycle comprises repeating steps (i) to (iii) with a plurality of isolation windows, wherein the plurality of isolation windows collectively span a mass-to-charge range of interest, optionally wherein the mass-to-charge range of interest corresponds to the mass-to-charge range of the mass spectrometer.

6. The method of any of claims 3 to 5, the method comprising repeating the data acquisition cycle.

**7.** The method of claim 6, wherein the method comprises introducing the sample in the mass spectrometer after chromatographic separation of the sample, and repeating the data acquisition cycle comprises repeating the data acquisition cycle at different retention times.

**8.** The method of claims 6 or 7, wherein repeating the data acquisition cycle comprises repeating the data acquisition cycle with at least one modified parameter selected from $[x^1_A, x^1_B]$ for one or more windows in a data acquisition cycle, $|x^1_A - x^1_B|$, $z1$, $z2$, $N$ and $z3$ and $M$, if present.

**9.** The method of any preceding claim, wherein N is between 2 and 6, preferably wherein N is 2, 3 or 4.

**10.** The method of any preceding claim, wherein $z1$ and $z2$ are each chosen to be between +1 and +12.

**11.** The method of any of claims 1 to 9, wherein $z1$ and $z2$ are each chosen to be between -1 and -12.

**12.** The method of any preceding claim, wherein the boundaries of the first isolation sub-window are defined as $m/z=[x^1_A, x^1_B]$ wherein $| x^1_A - x^1_B | = w$, and wherein:

> (a) $0 < w \leq 20$ Da, or
> *(b)* $0 < w \leq 10$ Da, or
> (c) w is 7, 8, 9, 10 or 11 Da, or
> (d) w is 1, 2, 3, 4 or 5 Da.

**13.** A mass spectrometer comprising:

> an ionisation source for generating ions from a sample;
> a mass selector for isolating precursor ions in a mass-to-charge (m/z) isolation window;
> an ion trap for storing precursor ions selected by the mass selector;
> a dissociation apparatus for fragmenting the precursor ions to generate fragment ions;
> a mass analyser for analysing the fragment ions; and
> a controller comprising a processor programmed to:

>> (i) control the mass selector to isolate precursor ions in an isolation window comprising N>=2 mass-to-charge isolation sub-windows and to store those precursor ions in the ion trap,
>> wherein the boundaries of a first isolation sub-window are defined as $m/z=[x^1_A, x^1_B]$ and the boundaries of the second and any subsequent isolation sub-window are defined as
$$m/z= \left[x^n_{A=\frac{(z1 \lor x^{n-1}_A - z1)+z2}{z2\lor}} , x^n_{B=\frac{(z1 \lor x^{n-1}_B - z1)+z2}{z2\lor}}\right]$$
where $z1$ is a first charge state and $z2$ is a second, different charge state, and $n=\{2,...,N\}$; and
>> (ii) control the ion trap to release the precursor ions in the ion trap for fragmentation and analysis.

**14.** The mass spectrometer of claim 13, wherein the controller processor is programmed to control the mass selector to isolate precursor ions in an isolation window comprising N>=2 mass-to-charge isolation sub-windows and to store those precursor ions in the ion trap, further comprising the controller processor being programmed to control the mass selector to isolate precursor ions in M>=1 additional mass-to-charge isolation sub-windows, and to store those additional precursor ions in the ion trap, wherein the boundaries of the first additional mass-to-charge isolation sub-window are defined as
$$m/z= \left[x^{m=1}_{A=\frac{(z1 \lor x^1_A - z1)+z3}{z3\lor}} , x^{m=1}_{B=\frac{(z1 \lor x^{n-1}_B - z1)+z3}{z3\lor}}\right],$$
and the boundaries of any subsequent additional mass-to-charge isolation sub-windows are defined as
$$m/z= \left[x^{m=2...M}_{A=\frac{(z1 \lor x^{m-1}_A - z1)+z3}{z3\lor}} , x^{m=2...M}_{B=\frac{(z1 \lor x^{m-1}_B - z1)+z3}{z3\lor}}\right],$$
where $z3$ is a third, different charge state, and $m=\{1,...,M\}$.

**15.** The mass spectrometer of claims 13 or 14, wherein the controller processor is further programmed to repeat steps (i) and (ii) with one or more isolation windows together forming a data acquisition cycle; and optionally repeat the data acquisition cycle.

Figure 1

200

ISOLATE PRECURSOR IONS IN
AN ISOLATION WINDOW

$$x^2_{A,B} = ((|z1|*x^1_{A,B}-z1)+z2)/|z2|$$

MS1 Mass Range (m/z)

...   ...   ...

$$x^3_{A,B} = ((|z1|*x^2_{A,B}-z1)+z2)/|z2|$$

210

FRAGMENT PRECURSOR IONS
TO GENERATE FRAGMENT IONS

220

MASS ANALYSE FRAGMENT
IONS

z1 precursor

z2 precursor

Common
fragments

Fragments from
z1 precursor

Fragments from
z2 precursor

Intensity

time

230

$$W = w + w^2 + w^3$$

time

w    $w^2$    $w^3$

Figure 2

MS1 mass range (m/z)

$x^1{}_A , x^1{}_B$

$x^2{}_A = ((|z1|*x^1{}_A\text{-}z1)+z2)/|z2|$

$x^2{}_B = ((|z1|*x^1{}_B\text{-}z1)+z2)/|z2|$

$x^3{}_A = ((|z1|*x^1{}_A\text{-}z1)+z3)/|z3|$

$x^3{}_B = ((|z1|*x^1{}_B\text{-}z1)+z3)/|z3|$

Figure 3

Figure 4

Figure 5A

Figure 5B

Figure 5C

Figure 5D

Figure 6

Figure 7

Figure 8

Figure 9

Europäisches
Patentamt

European
Patent Office

Office européen
des brevets

# EUROPEAN SEARCH REPORT

Application Number

EP 18 38 2384

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LUDOVIC C. GILLET ET AL: "Targeted Data Extraction of the MS/MS Spectra Generated by Data-independent Acquisition: A New Concept for Consistent and Accurate Proteome Analysis", MOLECULAR & CELLULAR PROTEOMICS, vol. 11, no. 6, 12 June 2012 (2012-06-12), XP55471043, US ISSN: 1535-9476, DOI: 10.1074/mcp.O111.016717 * abstract * * page 2 - page 3, paragraph 2; figures 1, 2, 6 * | 1-15 | INV. G01N33/68 |
| A | WO 2014/195783 A1 (DH TECHNOLOGIES DEV PTE LTD [SG]) 11 December 2014 (2014-12-11) * claims 1-15; figures 7, 8 * | 1-15 | |
| A | Francesco M. Mancuso ET AL: "Bioinformatic Approaches to Increase Proteome Coverage" In: "Gold Nanoparticles in Analytical Chemistry", 1 January 2014 (2014-01-01), Elsevier, XP55494601, ISSN: 0166-526X ISBN: 978-0-444-63285-2 vol. 63, pages 385-419, DOI: 10.1016/B978-0-444-62651-6.00017-9, * paragraph [02.3] * | 1-15 | |

TECHNICAL FIELDS
SEARCHED    (IPC)

G01N

-/--

The present search report has been drawn up for all claims

| Place of search | Date of completion of the search | Examiner |
|---|---|---|
| Munich | 25 July 2018 | Moreno de Vega, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 18 38 2384

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHIH-CHIANG TSOU ET AL: "DIA-Umpire: comprehensive computational framework for data-independent acquisition proteomics", NATURE METHODS, vol. 12, no. 3, 1 March 2015 (2015-03-01), pages 258-264, XP55494570, New York ISSN: 1548-7091, DOI: 10.1038/nmeth.3255 * page 258 - page 259 * | 1-15 | |
| A | WO 2016/125061 A1 (DH TECHNOLOGIES DEV PTE LTD [SG]) 11 August 2016 (2016-08-11) * claims 1-21; figures 9, 10 * | 1-15 | |
| A | EVA BORRÀS ET AL: "What is targeted proteomics? A concise revision of targeted acquisition and targeted data analysis in mass spectrometry", PROTEOMICS, vol. 17, no. 17-18, 1 September 2017 (2017-09-01), page 1700180, XP055494804, DE ISSN: 1615-9853, DOI: 10.1002/pmic.201700180 * the whole document * | 1-15 | TECHNICAL FIELDS SEARCHED (IPC) |

The present search report has been drawn up for all claims

| Place of search | Date of completion of the search | Examiner |
|---|---|---|
| Munich | 25 July 2018 | Moreno de Vega, C |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 18 38 2384

This annex lists the patent family members relating to the patent documents cited in the above-mentioned European search report.
The members are as contained in the European Patent Office EDP file on
The European Patent Office is in no way liable for these particulars which are merely given for the purpose of information.

25-07-2018

| Patent document cited in search report | | | Publication date | Patent family member(s) | | | Publication date |
|---|---|---|---|---|---|---|---|
| WO 2014195783 | A1 | | 11-12-2014 | CN | 105209907 | A | 30-12-2015 |
| | | | | EP | 3004871 | A1 | 13-04-2016 |
| | | | | JP | 2016524711 | A | 18-08-2016 |
| | | | | US | 2016109424 | A1 | 21-04-2016 |
| | | | | WO | 2014195783 | A1 | 11-12-2014 |
| WO 2016125061 | A1 | | 11-08-2016 | CA | 2975963 | A1 | 11-08-2016 |
| | | | | CN | 107210181 | A | 26-09-2017 |
| | | | | EP | 3254298 | A1 | 13-12-2017 |
| | | | | JP | 2018504607 | A | 15-02-2018 |
| | | | | US | 2018012742 | A1 | 11-01-2018 |
| | | | | WO | 2016125061 | A1 | 11-08-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CATHERMAN AD ; DURBIN KR ; AHLF DR ; EARLY BP ; FELLERS RT ; TRAN JC ; THOMAS PM ; KELLEHER NL.** Large-scale top-down proteomics of the human proteome: membrane proteins, mitochondria, and senescence. *Mol Cell Proteomics,* December 2013, vol. 12 (12), 3465-73
- **LUCA FORNELLI ; TIMOTHY K. TOBY ; LUIS F. SCHACHNER ; PETER F. DOUBLEDAY ; KRISTINA SRZENTI ; CAROLINE J. DEHART ; NEIL L. KELLEHER.** Top-down proteomics: Where we are, where we are going?. *Journal of Proteomics,* 20 March 2018, vol. 175, 3-4, https://doi.org/10.1016/j.jprot.2017.02.002
- **LUDOVIC C. GILLET ; PEDRO NAVARRO ; STEPHEN TATE ; HANNES RÖST ; NATHALIE SELEVSEK ; LUKAS REITER ; RON BONNER ; RUEDI AEBERSOLD.** Targeted Data Extraction of the MS/MS Spectra Generated by Data-independent Acquisition: A New Concept for Consistent and Accurate Proteome Analysis. *Molecular & Cellular Proteomics,* 18 January 2012, vol. 11
- **SKINNER, O S ; HAVERLAND, N A ; FORNELLI, L ; MELANI, R D ; VALE, DO L H F ; SECKLER, H S ; DOUBLEDAY, P F ; SCHACHNER, L F ; SRZENTIĆ, K ; KELLEHER, N L.** Top-down characterization of endogenous protein complexes with native proteomics. *Journal Article Nat. Chem. Biol.,* 2017
- **CHIH-CHIANG TSOU ; DMITRY AVTONOMOV ; BRETT LARSEN ; MONIKA TUCHOLSKA ; HYUNGWON CHOI ; ANNE-CLAUDE GINGRAS ; ALEXEY I NESVIZHSKII.** DIA-Umpire: comprehensive computational framework for data-independent acquisition proteomics. *Nature Methods,* 2015, vol. 12, 258-264